(19) Europäisches Patentamt
European Patent Office
Office européen des brevets

(11) EP 3 682 796 A1

(12) EUROPEAN PATENT APPLICATION

(43) Date of publication:
22.07.2020 Bulletin 2020/30

(51) Int Cl.:
A61B 5/00 (2006.01)          G16H 50/20 (2018.01)
G06F 16/33 (2019.01)

(21) Application number: 19152473.5

(22) Date of filing: 18.01.2019

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(71) Applicant: Koninklijke Philips N.V.
5656 AG Eindhoven (NL)

(72) Inventors:
• DE VRIES, Jan Johannes Gerardus
5656 AE Eindhoven (NL)

**Description**

FIELD OF THE INVENTION

This disclosure generally relates to an auditory verbal learning test (AVLT) in which a plurality of words are presented audibly to a subject and the subject has to repeat the words from memory. This disclosure relates in particular to a computer-implemented method, an apparatus and a computer program product for determining an updated set of words for use in an AVLT.

BACKGROUND OF THE INVENTION

Auditory verbal learning tests (AVLTs) are assessment tools frequently used in neuropsychological assessments to support the assessment of cognitive functions of an individual, such as verbal memory. These tests consist of a plurality (e.g. 15) words that are voiced (e.g. read or played) to the individual, and the individual has to repeat the words from memory (free recall), either immediately or with delay following the individual hearing the words. Several trials are used to measure learning, recall and recognition. In clinical practice, AVLT assessments are often repeated over time to measure changes in the individual's cognitive function over time.

AVLTs are carefully designed, i.e. based on the frequency and familiarity of words and scientifically validated (i.e. reliable and valid); thus the words used in the AVLT can therefore not be chosen completely at random.

However, AVLTs are known to have practice effects, i.e. an individual's performance can improve because they remember one or more of the words in the test from the previous assessment, or previous assessments. This can compromise the reliability and validity of the AVLT as the result will be dependent on aspects other than the short term verbal memory of the individual that the test is designed for.

Therefore, there is a need for a means to determine an updated set of words for an AVLT to help mitigate practice effects associated with an individual repeating an AVLT with the same set of words, while ensuring that the words maintain their role in supporting the assessment of cognitive functions of the individual.

SUMMARY OF THE INVENTION

According to a first specific aspect, there is provided a computer-implemented method of determining an updated set of words for use in an auditory verbal learning test, AVLT, on a first subject, the method comprising: receiving, by a processing unit, an initial set of words for use in an AVLT, wherein the initial set comprises a predetermined number of a plurality of words stored in a database; processing, by the processing unit, the initial set to determine feature values for the initial set; extracting, by the processing unit, one or more words from the database based on a desired level of similarity between the feature values associated with the one or more extracted words and the feature values of the initial set; and selecting, by the processing unit, one or more of the extracted words to include in an updated set of words for use in the AVLT for the first subject. Thus, an updated set of words can be generated which can help mitigate practice effects associated with an individual repeating an AVLT with the same set of words. In addition, the desired level of similarity enables the difficulty level of the AVLT to be increased, decreased or maintained as desired.

In some embodiments, the feature values for the initial set comprise a respective concreteness score for each word that represents a level of abstractness of a concept represented by the word, a number of characters in the word, a number of vowels and/or a number of consonants in the word, a number of syllables in the word, an originating language or a frequency of use of the word in text. These feature values can provide an indication of a difficulty level associated with recalling the word in an AVLT, enabling the desired level of similarity to be used to adjust or maintain a difficulty level of the AVLT using the updated set of words.

In some embodiments, the feature values for the initial set comprises a respective feature value for each word in the initial set, and wherein the step of extracting comprises, for at least one word in the initial set, extracting one or more words from the database having a respective feature value that is related to the feature value of said at least one word in the initial set based on the desired level of similarity.

In alternative embodiments, the step of extracting comprises, for at least one word in the initial set, extracting one or more words from the database by: calculating a probability distribution as $P\_w = (p\_w,x) / \sum(p\_w,x)$, where $p\_w, x = 1/d(c\_w,c\_x)$, $c\_w$ is the feature value for a word $w$ in the initial set, $c\_x$ is the feature value for a word $x$ in the database, where $x \neq y$, and $d(c\_w,c\_x)$ is a distance measure representing a distance between $c\_w$ and $c\_x$ subject to a bias value $\delta$, where the bias value $\bar{\delta}$ is indicative of a desired offset in feature values; and randomly extracting one or more words from the database using the probability distribution. Randomly extracting one or more words from the database introduces variety into the words in the updated set, and in subsequent updated sets.

In some embodiments, the feature values for the initial set comprise distances between pairs of words in the initial set. Distances are a useful way to measure or represent distances between words. In these embodiments, the step of processing the initial set to determine the feature values can comprise determining the distance between each pair of words using an ontology. Ontologies are a common way to describe similarity between words or concepts. In these embodiments, the step of extracting can comprise, for at least one word in the initial set, extracting one or more words from the database having a maximum distance with respect to the other words in the initial set based on the desired level of similarity. Alternatively, in these embodiments, the step of extracting can comprise forming an initial weighted graph from the words in the initial set and the distances, wherein the distances form weights along edges of the graph and the words form vertices of the graph; finding the minimal spanning tree of the initial weighted graph; forming a database weighted graph from the words in the database and distances between each pair of words in the database; identifying a subtree in the database weighted graph having the desired level of similarity to the minimal spanning tree of the initial weighted graph; and extracting one or more words from the database according to the identified subtree in the database weighted graph. The database weighted graph is a convenient mathematical approach to represent the data structure and allow for the application of various algorithms to do the computation. In these embodiments, the desired level of similarity can be a required difference in a number of vertices between a subtree in the database weighted graph and the minimal spanning tree of the initial weighted graph and a required difference in a distribution of weighted edges between a subtree in the database weighted graph and the minimal spanning tree of the initial weighted graph.

In some embodiments, the method further comprises: storing, in a memory unit, a results database comprising results for AVLTs previously-performed by a plurality of subjects, and respective user profiles for the plurality of subjects, wherein the results indicate whether the words in the AVLTs were successfully recalled by the subject. In these embodiments, the results database can comprise one or more results for the first subject, and wherein the method further comprises determining the desired level of similarity based on the one or more results for the first subject. This has the advantage that the updated set of words can be generated with the difficulty level of the AVLT being adapted or maintained based on previous performance(s) of the AVLT by the first subject. In these embodiments, the method can further comprise: analyzing the stored results and respective user profiles to determine a relationship between successful recall of a word and user profiles. In these embodiments, the step of extracting can comprise: extracting one or more words from the database based on the desired level of similarity, a first user profile of the first subject and the determined relationship. In these embodiments, the step of extracting can comprise: using an ontology to identify one or more words in the database for the first subject based on a first user profile for the first subject; and extracting one or more words from the database based on the desired level of similarity and the ontology-identified one or more words. In these embodiments, a user profile can indicate one or more of sociodemographic, cultural and behavioral characteristic of the respective subject. This provides the advantage that the difficulty level of the AVLT can be adapted or maintained using words that are appropriate for a sociodemographic, cultural and/or behavioral characteristic of the first subject, in other words tailoring the AVLT to the characteristics of the first subject.

According to a second aspect, there is provided a computer-implemented method of administering an auditory verbal learning test, AVLT, to a first subject, the method comprising: determining an updated set of words for use in an AVLT according to the first aspect or any embodiment thereof; and outputting, via a user interface, the updated set of words to the first subject.

According to a third aspect, there is provided a computer program product comprising a computer readable medium having computer readable code embodied therein, the computer readable code being configured such that, on execution by a suitable computer or processor, the computer or processor is caused to perform the method according to the first aspect or any embodiment thereof, or according to the second aspect.

According to a fourth specific aspect, there is provided an apparatus for determining an updated set of words for use in an auditory verbal learning test, AVLT, on a first subject, the apparatus comprising a processing unit wherein the processing unit (8) is configured to: receive an initial set of words for use in an AVLT, wherein the initial set comprises a predetermined number of a plurality of words stored in a database; process the initial set to determine feature values for the initial set, extract one or more words from the database based on a desired level of similarity between feature values associated with the one or more extracted words and the feature values of the initial set; and select one or more of the extracted words to include in an updated set of words for use in the AVLT for the first subject. Thus, an updated set of words can be generated which can help mitigate practice effects associated with an individual repeating an AVLT with the same set of words. In addition, the desired level of similarity enables the difficulty level of the AVLT to be increased, decreased or maintained as desired.

In some embodiments, the feature values for the initial set comprise a respective concreteness score for each word that represents a level of abstractness of a concept represented by the word, a number of characters in the word, a number of vowels and/or a number of consonants in the word, a number of syllables in the word, an originating language or a frequency of use of the word in text. These feature values can provide an indication of a difficulty level associated with recalling the word in an AVLT, enabling the desired level of similarity to be used to adjust or maintain a difficulty level of the AVLT using the updated set of words.

In some embodiments, the feature values for the initial set comprises a respective feature value for each word in the initial set, and wherein the processing unit is configured to extract one or more words from the database by, for at least one word in the initial set, extracting one or more words from the database having a respective feature value that is related to the feature value of said at least one word in the initial set based on the desired level of similarity.

In alternative embodiments, the processing unit is configured to, for at least one word in the initial set, extract one or more words from the database by: calculating a probability distribution as $P\_w = (p\_w,x) / \sum(p\_w,x)$, where $p\_w, x = 1/d(c\_w,c\_x)$, $c\_w$ is the feature value for a word w in the initial set, $c\_x$ is the feature value for a word x in the database, where $x \neq y$, and $d(c\_w,c\_x)$ is a distance measure representing a distance between $c\_w$ and $c\_x$ subject to a bias value $\delta$, where the bias value $\delta$ is indicative of a desired offset in feature values; and randomly extracting one or more words from the database using the probability distribution. Randomly extracting one or more words from the database introduces variety into the words in the updated set, and in subsequent updated sets.

In some embodiments, the feature values for the initial set comprise distances between pairs of words in the initial set. Distances are a useful way to measure or represent distances between words. In these embodiments, the processing unit can be configured to process the initial set to determine the feature values by determining the distance between each pair of words using an ontology. Ontologies are a common way to describe similarity between words or concepts. In these embodiments, the processing unit can be configured to, for at least one word in the initial set, extract one or more words from the database having a maximum distance with respect to the other words in the initial set based on the desired level of similarity. Alternatively, in these embodiments, the processing unit can be configured to extract the one or more words by: forming an initial weighted graph from the words in the initial set and the distances, wherein the distances form weights along edges of the graph and the words form vertices of the graph; finding the minimal spanning tree of the initial weighted graph; forming a database weighted graph from the words in the database and distances between each pair of words in the database; identifying a subtree in the database weighted graph having the desired level of similarity to the minimal spanning tree of the initial weighted graph; and extracting one or more words from the database according to the identified subtree in the database weighted graph. The database weighted graph is a convenient mathematical approach to represent the data structure and allow for the application of various algorithms to do the computation. In these embodiments, the desired level of similarity can be a required difference in a number of vertices between a subtree in the database weighted graph and the minimal spanning tree of the initial weighted graph and a required difference in a distribution of weighted edges between a subtree in the database weighted graph and the minimal spanning tree of the initial weighted graph.

In some embodiments, a memory unit can be further configured to store a results database comprising results for AVLTs previously-performed by a plurality of subjects, and respective user profiles for the plurality of subjects, wherein the results indicate whether the words in the AVLTs were successfully recalled by the subject. In these embodiments, the results database can comprise one or more results for the first subject, and wherein the processing unit can be configured to determine the desired level of similarity based on the one or more results for the first subject. This has the advantage that the updated set of words can be generated with the difficulty level of the AVLT being adapted or maintained based on previous performance(s) of the AVLT by the first subject. In these embodiments, the processing unit can be configured to: analyze the stored results and respective user profiles to determine a relationship between successful recall of a word and user profiles. In these embodiments, the processing unit can be configured to extract one or more words from the database based on the desired level of similarity, a first user profile of the first subject and the determined relationship. In these embodiments, the processing unit can be configured to extract the one or more words by: using an ontology to identify one or more words in the database for the first subject based on a first user profile for the first subject; and extracting one or more words from the database based on the desired level of similarity and the ontology-identified one or more words. In these embodiments, a user profile can indicate one or more of sociodemographic, cultural and behavioral characteristic of the respective subject. This provides the advantage that the difficulty level of the AVLT can be adapted or maintained using words that are appropriate for a sociodemographic, cultural and/or behavioral characteristic of the first subject, in other words tailoring the AVLT to the characteristics of the first subject.

In some embodiments, the apparatus is further for administering the AVLT to the first subject, and the apparatus further comprises a user interface that is for outputting the updated set of words to the first subject.

These and other aspects will be apparent from and elucidated with reference to the embodiment(s) described hereinafter.

BRIEF DESCRIPTION OF THE DRAWINGS

Exemplary embodiments will now be described, by way of example only, with reference to the following drawings, in which:

Fig. 1 is a block diagram illustrating an apparatus according to an exemplary embodiment; and
Fig. 2 is a flow chart illustrating a method according to an exemplary embodiment.

DETAILED DESCRIPTION OF EMBODIMENTS

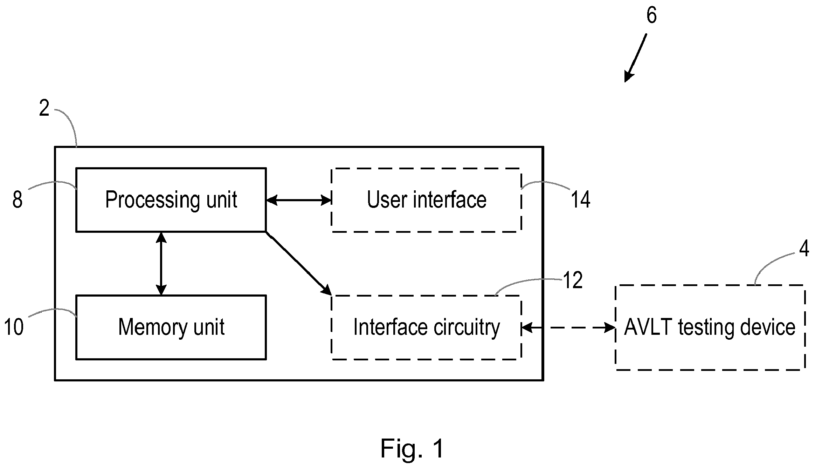

Fig. 1 shows a block diagram of an exemplary apparatus 2 that can be used for determining an updated set of words for use in an auditory verbal learning test (AVLT). The updated set of words is to be used in an AVLT for a 'first subject', and the 'first subject' referenced herein is the person or individual that is to take part in the AVLT using the updated set of words. A 'user' of the apparatus 2 is typically a healthcare professional that wishes to conduct an AVLT.

The apparatus 2 may be configured to output the updated set of words to the user of the apparatus 2 (e.g. a healthcare professional) so that the user/person can read out the updated set of words to the first subject and record the words recited by the first subject. Alternatively, the apparatus 2 can be connected to an AVLT testing device 4 that conducts the AVLT with the first subject (e.g. by automatically reading out or playing the set of words to the first subject via a loudspeaker and recording the words recited by the first subject using a microphone). In this case the apparatus 2 and AVLT testing device 4 form a system 6 for providing AVLT tests to a first subject (and potentially other subjects). In other embodiments, the apparatus 2 can both determine the updated set of words, and conduct the AVLT with the first subject (in which case the first subject can also be considered a user of the apparatus 2).

The apparatus 2 is an electronic device that comprises a processing unit 8 and a memory unit 10. The processing unit 8 is configured or adapted to control the operation of the apparatus 2 and to implement the techniques described herein for determining an updated set of words to use in an AVLT.

The processing unit 8 can be configured to execute or perform the methods described herein. The processing unit 8 can be implemented in numerous ways, with software and/or hardware, to perform the various functions described herein. The processing unit 8 may comprise one or more microprocessors or digital signal processor (DSPs) that may be programmed using software or computer program code to perform the required functions and/or to control components of the processing unit 8 to effect the required functions. The processing unit 8 may be implemented as a combination of dedicated hardware to perform some functions (e.g. amplifiers, pre-amplifiers, analog-to-digital convertors (ADCs) and/or digital-to-analog convertors (DACs)) and a processor (e.g., one or more programmed microprocessors, controllers, DSPs and associated circuitry) to perform other functions. Examples of components that may be employed in various embodiments of the present disclosure include, but are not limited to, conventional microprocessors, DSPs, application specific integrated circuits (ASICs), and field-programmable gate arrays (FPGAs).

The processing unit 8 is connected to a memory unit 10 that can store data, information and/or signals for use by the processing unit 8 in controlling the operation of the apparatus 2 and/or in executing or performing the methods described herein. For example, the memory unit 10 can store any of, an initial set of words to use in an AVLT, a database of words, and/or feature values for words in the initial set and/or database. In some implementations the memory unit 10 stores computer-readable code that can be executed by the processing unit 8 so that the processing unit 8, in conjunction with the memory unit 10, performs one or more functions, including the methods described herein. The memory unit 10 can comprise any type of non-transitory machine-readable medium, such as cache or system memory including volatile and non-volatile computer memory such as random access memory (RAM), static RAM (SRAM), dynamic RAM (DRAM), read-only memory (ROM), programmable ROM (PROM), erasable PROM (EPROM), and electrically erasable PROM (EEPROM), and the memory unit 10 can be implemented in the form of a memory chip, an optical disk (such as a compact disc (CD), a digital versatile disc (DVD) or a Blu-Ray disc), a hard disk, a tape storage solution, or a solid state device, including a memory stick, a solid state drive (SSD), a memory card, etc.

In some embodiments, the apparatus 2 can include interface circuitry 12 for enabling a data connection to and/or data exchange with other devices, including any one or more of servers, databases, user devices, and one or more AVLT testing devices 4. The data to be exchanged or sent to the other devices can include an updated set of words for use in an AVLT. The connection may be direct or indirect (e.g. via the Internet), and thus the interface circuitry 12 can enable a connection between the apparatus 2 and a network, such as the Internet, via any desirable wired or wireless communication protocol. For example, the interface circuitry 12 can operate using WiFi, Bluetooth, Zigbee, or any cellular communication protocol (including but not limited to Global System for Mobile Communications (GSM), Universal Mobile Telecommunications System (UMTS), Long Term Evolution (LTE), LTE-Advanced, etc.). In the case of a wireless connection, the interface circuitry 12 (and thus apparatus 2) may include one or more suitable antennas for transmitting/receiving over a transmission medium (e.g. the air). Alternatively, in the case of a wireless connection, the interface circuitry 12 may include means (e.g. a connector or plug) to enable the interface circuitry 12 to be connected to one or more suitable antennas external to the apparatus 2 for transmitting/receiving over a transmission medium (e.g. the air). The interface circuitry 12 is connected to the processing unit 8.

In some embodiments, the apparatus 2 comprises a user interface 14 that includes one or more components that enables a user of apparatus 2 (e.g. a healthcare professional or the first subject) to input information, data and/or commands into the apparatus 2, and/or enables the apparatus 2 to output information or data to the user of the apparatus 2 (e.g. a healthcare professional or the first subject). As used herein, the 'user' of the apparatus can be a person, such as a neuropsychologist, that would like to determine if a test subject (referred to as a 'subject' or 'first subject' herein) is malingering. In embodiments where the apparatus 2 includes or is part of a testing device, the subject can also be considered as a user of the apparatus 2.

The user interface 14 can comprise any suitable input component(s), including but not limited to a keyboard, keypad, one or more buttons, switches or dials, a mouse, a track pad, a touchscreen, a stylus, a camera, a microphone, etc., and the user interface 14 can comprise any suitable output component(s), including but not limited to a display screen, one or more lights or light elements, one or more loudspeakers, a vibrating element, etc.

In embodiments where the apparatus 2 is to be used to conduct the AVLT with the first subject, the user interface 14 can include a loudspeaker for verbally outputting the words in the updated set to the first subject. The loudspeaker (or other output component) can be used to provide other instructions regarding the test to the first subject (e.g. an instruction to start reciting the words). The user interface 14 can also include a microphone to record the words spoken by the first subject. The results of the AVLT may be assessed by a healthcare professional, for example by listening to a recording of the words spoken by the first subject. Alternatively, the processing unit 8 may be configured to process the signal from the microphone to identify the words spoken by the first subject, compare the identified words to the words in the updated set, and to output a score or other indicator (including a list of the words correctly recited and/or a list of the words that were missed) of the result of the AVLT by the first subject. Techniques for the processing of a microphone signal to identify words spoken by the first subject are outside the scope of this disclosure, but those skilled in the art will be aware of suitable techniques that can be used.

The apparatus 2 can be any type of electronic device or computing device. For example the apparatus 2 can be, or be part of, a server, a computer, a laptop, a tablet, a smartphone, a smartwatch, etc. In some implementations, for example where the apparatus 2 is also used as the AVLT test device for presenting the AVLT to the first subject, the apparatus 2 can be an apparatus that is present or used in the home or care environment of the first subject. In other implementations, the apparatus 2 is an apparatus that is remote from the first subject, and remote from the home or care environment of the first subject.

It will be appreciated that a practical implementation of an apparatus 2 may include additional components to those shown in Fig. 1. For example the apparatus 2 may also include a power supply, such as a battery, or components for enabling the apparatus 2 to be connected to a mains power supply.

As noted above, there a problem with AVLTs is that the results of the tests can be affected by so-called practice effects, whereby an individual's performance can improve with subsequent repetitions of the AVLT because they have remembered one or more of the words in the test from the previous assessment. This can compromise the reliability and validity of the AVLT as the result of the AVLT will be dependent on aspects other than the short term verbal memory of the individual that the test is designed for (e.g. one aspect that can affect the result is the long term verbal memory of the first subject). The list or set of words used in AVLTs is carefully designed, and so an updated set of words to use in the AVLT cannot be chosen completely at random.

Therefore, the techniques described herein provide a way to determine an updated set of words for an AVLT to help mitigate practice effects associated with an individual repeating an AVLT with the same set of words. In particular the techniques described herein enable one or more words in an initial set of words for an AVLT to be replaced by different words to form an updated set. According to particular embodiments, the one or more words can be replaced with words that make the AVLT easier (e.g. it is easier for a subject to recall them), harder (e.g. it is harder for a subject to recall them), or generally the same difficulty (e.g. it is generally not easier or harder for a subject to recall them). These embodiments can be used to tailor the AVLT to the ability of a particular first subject. According to other embodiments (which can be used separately or in combination with those above), the one or more words can be replaced to increase or decrease the suitability of the words in the updated set to the sociodemographic and/or cultural background of the first subject. For example, words can carry different meanings or associations depending on a subject's sociodemographic and/or cultural background, and so certain words may not be appropriate or too easy for a particular subject to recall. According to other embodiments (which can be used separately or in combination with those above), the one or more words can be replaced to increase or decrease the suitability of the words in the updated set to the ability (e.g. based on previous AVLT performance) and/or personal interests of the first subject. For example, words associated with a personal interest of the subject may be much easier for the subject to remember, in which case that word or words can be replaced in the updated set.

Thus, the embodiment described herein provide that an updated set of words can be provided to dynamically adapt the difficulty level of the AVLT based on the first subject's previous AVLT performance(s), sociodemographic/cultural background and/or personal interests. Dynamically adapting the difficulty level in this way may not only improve the outcomes of the test (e.g. in avoiding the practice effects), but it can also improve the engagement of the first subject in performing the test, which is particularly useful when repeated testing is required.

Briefly, according to the techniques described herein for determining an updated set of words for an AVLT, an initial set of words for an AVLT is obtained, and the method outputs an updated set of words for an AVLT in which one or more of the words in the initial set have been changed. Additional inputs to the method to enable the selection of suitable words for the updated set differs according to the embodiment, which are outlined below, but can include characteristics of words (e.g. a measure of concreteness of the concept represented by the word), semantic relationships of words (such as ontologies), corpora representing natural language, and sociodemographic, cultural and/or behavioral (including previous AVLT performance) information for the first subject. As noted above, it is possible for each of the embodiments to generate the updated word set so that the updated word set provides an AVLT of the same difficulty or generally the same difficulty, or provides an AVLT with a lower or higher difficulty level. In some embodiments, after the set of words is updated and used in an AVLT for the first subject, the first subject's performance in the AVLT using the updated set can be assessed and used to determine further updates to the set. This can provide a feedback loop that has the aim of balancing the first subject's performance by optimizing the difficulty level of the AVLT. For example, the performance by the first subject in the previous attempts at the AVLT can be used to determine whether the AVLT should be made more difficult or easier by comparing the performance to a preset performance level (e.g. an optimum performance level). This might mean that, for example, if the performance of the first subject is above the optimal performance level, the next AVLT should be made more difficult. By achieving a better balance between the first subject's performance/ability with the difficulty level of the AVLT, it will be possible to better judge changes in performance (over time) for subjects that are at the extreme ends of the performance scale (i.e. close to perfect performance or close to zero performance). This improved balance will also enhance a subject's engagement in repeating the AVLT over time (with different word sets) because the AVLT remains sufficiently challenging for that subject.

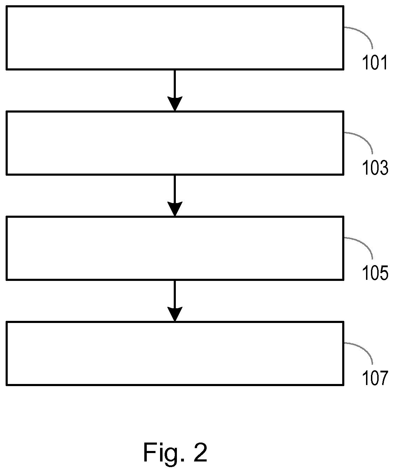

The flow chart in Fig. 2 illustrates an exemplary method according to the techniques described herein. One or more of the steps of the method can be performed by the processing unit 8 in the apparatus 2, in conjunction with the memory unit 10 as appropriate. The processing unit 8 may perform the one or more steps in response to executing computer program code that can be stored on a computer readable medium, such as, for example, the memory unit 10, or a separate memory device or storage medium. In particular, the processing unit 8 can perform any one or more of steps 101, 103, 105 and 107 below.

In a first step, step 101, an initial set of words for use in an AVLT is received. The initial set of words comprises a predetermined number of a plurality of words stored in a database. In some AVLTs, the set comprises 15 different words, but it will be appreciated that AVLTs can use more or less than 15 words as required. The initial set of words may be a standard set of words for an AVLT, for example a set of words for an AVLT described in a scientific publication or standardized according to a medical protocol. Alternatively, the initial set of words can be any set of words, including a set of words that has previously been updated or determined according to the techniques described herein. The database typically stores a large number of words, much larger than the number of words used in each AVLT word set (for example the database can include a number of words that is one or more orders of magnitude higher than the number of words used in a set of words for the AVLT). The database can be stored in the memory unit 10, or a separate memory unit.

Next, in step 103, the initial set of words are processed to determine feature values for the initial set. As described in more detail below, in some embodiments the feature values for the initial set can be respective feature values for each word in the initial set. In these embodiments, a feature value for a word can be a concreteness score that represents a level of abstractness of a concept represented by the word (where concreteness and abstractness are the inverse of each other). Alternative feature values could be the number of characters in the word (otherwise referred to as the word length), the number of vowels and/or number of consonants in the word, the number of syllables in the word, the originating language of the word, or the frequency of use of the word in some text (e.g. a newspaper or magazine, or a range of publications). In alternative embodiments, the feature values for the initial set can be measures of a distance between pairs of words in the initial set. In some embodiments, the distance measure is a measure of the semantic distance between the pair of words, i.e. a measure of the difference in the semantic meaning of the words in the pair. Such semantic meaning differences can be provided by an ontology.

In some embodiments, step 103 can comprise determining multiple feature values for each word. For example step 103 can comprise determining a concreteness score and a word length, or a concreteness score for a word and semantic distances for that word with the other words in the initial set. Subsequent steps of the method use the multiple feature values for each word.

In step 105, one or more words are extracted from the database. The one or more words to be extracted from the database are determined based on a desired level of similarity between feature values associated with the one or more extracted words and the feature values of the initial set. As described in more detail below, the desired level of similarity can indirectly represent a desired adjustment in the difficulty of AVLT when using one or more of the extracted words in an AVLT. For example, if it is desired to maintain the difficulty level of an AVLT based on the initial set of words, the desired level of similarity may be high so that the first subject experiences a similar difficulty level when performing an AVLT using an updated set that includes one or more of the extracted words that have a similar feature value. As another example, if it is desired to make the AVLT easier than with the initial set of words, the desired level of similarity can be low (with an indication that the feature values associated with the words to be extracted should be higher or lower as appropriate to provide a word that is 'easier' to recall), and vice versa to make the AVLT harder than with the initial set of words.

Finally, in step 107, one or more of the extracted words are selected for inclusion in an updated set of words for use in the AVLT for the first subject. This step can comprise (or be followed by a separate step of) substituting one or more of the words in the initial set with one or more of the extracted words to form the updated set. This substitution can be understood as directly 'swapping' or 'exchanging' a word from the initial set with an extracted word, or alternatively as adding one or more of the extracted words and potentially one or more of the words in the initial set to an (initially) 'empty' set of words to form an updated set that includes the same number of words as the initial set. In both implementations, it will be appreciated that the updated set of words will include one or more of the extracted words, and (where not all of the words in the initial set are substituted or one or more words in the initial set are added to the empty set) one or more of the words from the initial set. For example, for an initial set of fifteen words, the first, fifth and tenth words in the initial set can be replaced by respective extracted words, so that the updated set includes the three extracted words and the other twelve words from the initial set. In the alternative approach that starts with an empty set, the same set can be formed by adding the three extracted words and the other words from the initial set.

In some embodiments, a particular word that was extracted from the database can be included in the updated set on the basis of the feature value of the extracted word in comparison with the feature value of a word in the initial set (e.g. the word in the initial set that the extracted word is to replace). For example, the feature value of a particular word or word pair in the initial set may have resulted in one or more words being extracted from the database (based on the feature value of the extracted word(s) and the desired level of similarity). The particular word or one of the words in the word pair in the initial set can therefore be replaced by one of those extracted words (or alternatively the extracted word and the other word in the word pair in the initial set can be added to the updated set). In the event that multiple words are extracted from the database for a particular word or word pair, the extracted word to use in the updated set can be selected at random.

In some embodiments, the method can further comprise the step of outputting the updated set of words, or outputting information about the updated set of words (for example information identifying the words that are in the updated set). The updated set of words or information about the updated set of words can be output to the AVLT testing device 4 so that they can be presented to the first subject.

In a first set of embodiments, the feature values for the initial set of words comprise respective feature values for each word in the initial set. In these embodiments, step 105 can comprise, for at least one word in the initial set, extracting one or more words from the database having a respective feature value that is related to the feature value of said at least one word in the initial set based on the desired level of similarity. This extraction can be performed for one, a plurality, or all of the words in the initial set. As noted above, in some embodiments, the feature value could be the number of characters in the word (otherwise referred to as the word length), the number of vowels and/or number of consonants in the word, the number of syllables in the word, the originating language of the word or the frequency of use of the word in some text (e.g. a newspaper or magazine, or a range of publications).

Also as noted above, another example of a feature value for an individual word is a concreteness score that represents a level of abstractness of a concept represented by the word (where concreteness and abstractness are the inverse of each other). It is known that words with a higher abstractness (e.g. freedom, happiness) are more difficult to memorize than words with a low abstractness (e.g. house, dog). As such, changing the abstractness of the words in the updated set relative to the initial set AVLT can change the difficulty level of the AVLT.

A concreteness score can be derived for each word in step 103, or, in some embodiments, in step 103 the concreteness score for each word in the initial set can be obtained from a separate database of concreteness/abstractness that provides concreteness scores for a plurality of words (or alternatively the database stored in step 101 can include the concreteness scores for the words in the database). Such a database is described in the paper "Concreteness ratings for 40 thousand generally known English word lemmas" by Marc Brysbaert et. al (and which can be found at: http://crr.ugent.be/papers/Brysbaert_Warriner_ Kuperman_BRM_Concreteness_ratings.pdf. The scores in this database describe per word how abstract vs concrete the concept represented by the word is, and it has been compiled through human (manual) rating.

In step 105, the concreteness score for the words in the database can also be looked up in the separate database of concreteness/abstractness (unless the database referenced in step 101 already includes this information), and one or more words having the desired level of similarity between their concreteness score and the concreteness score of the words in the initial set are extracted. Thus, to generally maintain the same level of difficulty, for a given word in the initial set, one or more words should be extracted that have a similar concreteness score. To generally increase the level of difficulty, for a given word in the initial set, one or more words should be extracted that have a lower concreteness score (i.e. they are more abstract) than the word in the initial set (in this case the desired level of similarity is that the extracted word should have a lower concreteness score). To generally decrease the level of difficulty, for a given word in the initial set, one or more words should be extracted that have a higher concreteness score (i.e. they are less abstract) than the word in the initial set (in this case the desired level of similarity is that the extracted word should have a higher concreteness score).

In particular embodiments of step 105, the extraction of the one or more words can be performed by choosing a random word from the database stored in step 101 using the inverse of the distance between the concreteness score of the word in the initial set and a word in the database as weights in a probability distribution. Thus, for at least one word in the initial set, a probability distribution is calculated for the words in the database as:

$$P\_w = (p\_w,x) / \Sigma(p\_w,x) \qquad\qquad (1)$$

where $p\_w,x = 1/d(c\_w,c\_x)$, $c\_w$ is the feature value (e.g. concreteness score) for a word w in the initial set, $c\_x$ is the feature value (e.g. concreteness score) for a word x in the database stored in step 101, where $x \neq y$ (so the words are not the same), and $d(c\_w,c\_x)$ is a distance measure representing a distance between $c\_w$ and $c\_x$ subject to a bias value $\delta$, where the bias value $\delta$ is dependent on the desired level of similarity. The distance measure d can be the absolute difference, $d(c\_w,c\_x) = |(c\_w - c\_x)|$, or the squared Euclidean distance $d(c\_w,c\_x) = (c\_w - c\_x)^2$, or any other suitable distance measure. In order to manipulate the difficulty level, the distance measure can be biased using the bias value $\delta$. For example the biased distance measure d can be given by $d(c\_w,c\_x) = |((c\_w - c\_x) - \delta)|$. The bias value $\delta$ effectively represents the desired offset in feature value (e.g. difficulty level), and the bias value $\delta$ should be 0 or near 0 to maintain the same feature values (e.g. level of difficulty). To increase the feature value (e.g. difficulty) the bias value $\delta$ should be larger than 0 and to decrease the feature value (e.g. difficulty), the bias value $\delta$ should be smaller than 0.

The probability distribution is then used to randomly extract one or more words from the database. For example, the unit interval can be divided into intervals corresponding to all values from the probability distribution P_w (i.e. if there are N words covered by P_w, the unit interval is divided into N segments where each segment has length p_w,x). A uniformly distributed random number can be drawn from the unit interval, the interval in which the random number falls can be identified, and the extracted word corresponding to the identified interval can be selected.

In the event that a word in the initial set is not present in the database, or in case it is desired to extend the database, it is possible to extrapolate the concreteness values for the words in the database to other words. For example, this can be done by using word symmetry values obtained from semantic structures such as ontologies that describe how similar word concepts are. Thus, for all words in the database, their symmetry can be looked up in the ontology, and these symmetry values can be used as weights in a function that derives an estimated concreteness value for the new word from the concreteness values of existing words in the database. This function could, in some embodiments, be a weighted average.

It will be appreciated that the above approach for extracting words according to the concreteness values can be applied to any embodiment where the feature value reflects a level of difficulty, such as, the number of characters in the word, the number of vowels and/or number of consonants in the word, the number of syllables in the word, the originating language or the frequency of use of the word in some text. For example, in step 103 the feature value for the words in the initial set can be derived from the word or obtained from a database. In step 105 the feature values for the words in the database can also be looked up, and one or more words having the desired level of similarity between their feature value and the feature value of the words in the initial set are extracted. Thus, to generally maintain the same level of difficulty, for a given word in the initial set, one or more words should be extracted that have a similar feature value. To generally increase the level of difficulty, for a given word in the initial set, one or more words should be extracted that have a feature value indicative of higher difficulty (e.g. a word with a higher number of characters, a word with a higher number of vowels and/or consonants, a word with a higher number of syllables, a word originating from a different language or a word with a lower frequency of use of the word in some text). To generally decrease the level of difficulty, for a given word in the initial set, one or more words should be extracted that have a feature value indicative of lower difficulty (e.g. a word with a lower number of characters, a word with a lower number of vowels and/or consonants, a word with a lower number of syllables, a word native to the language or a word with a higher frequency of use of the word in some text). It will be appreciated that the extraction of the one or more words in step 105 can be performed by choosing a random word from the database using the inverse of the distance between the feature value of the word in the initial set and a word in the database as weights in a probability distribution, as described above for the concreteness value embodiment.

In a second set of embodiments, the feature values for the initial set can be measures of a distance between pairs of words in the initial set. For example, for a first word in the initial set and a second word in the initial set, there is a feature value representing the distance between those two words. There is also a distance measure between the first word and a third word in the initial set, and so on. In some embodiments, the distance measure is a measure of the semantic distance between the pair of words, i.e. a measure of the difference in the semantic meaning of the words in the pair.

Thus, in these embodiments, the words in the set used for the AVLT use a methodology describing the word space from which the similarity and/or distance between words can be established, e.g. using a semantic distance known from an ontology (where it will be appreciated that different ontologies can provide different semantic distances for a particular pair of words). It is known that words that are semantically related (e.g. bed, pillow, dream) are easier to memorize than words that are not semantically related (e.g. tree, door, intention). By changing the semantic distance of the words in the initial set for the AVLT, the difficulty level of the AVLT can be adapted. In these embodiments, step 103 can comprise determining the semantic distance between each pair of words using an ontology. In some embodiments, step 105 can comprise, for at least one word in the initial set, extracting one or more words from the database having a maximum semantic distance with respect to the other words in the initial set based on the desired level of similarity.

The set of 'pairwise' distances (whether semantic distances or otherwise) can be represented as an undirected weighted graph, where the weights along edges are formed by the distances and the vertices are formed by the words. By finding the minimal spanning tree of this graph, it is possible to obtain the closest (e.g. in terms of semantics if semantic distance is used) set of matching word pairs representing all words in the initial set, if it is desired to replace the initial set with an updated set that provides a similar level of difficulty.

For the words in the database, a graph can be constructed containing all of the words from the database as vertices, and distances of the word-pairs as weighted edges. An updated set of words can be determined in steps 105 and 107 by finding a tree in this graph that has the same number of vertices and a set of weighted edges that represents the distribution of weighted edges from the minimal spanning tree of the initial set. This can be done to iterate over all possible trees of a given size (for example as described at https://stackoverflow.com/questions/5692286/find-all-subtrees-of-size-n-in-an-undirected-graph), or by applying a more advanced search strategy given the search space consisting of all possible trees of said given size.

By applying a similar technique as explained above with respect to the first set of embodiments, some randomness can be introduced by selecting from the words extracted in step 105 using the inverse of some distance function to the (minimal spanning tree representing the) original set of words as probabilities. In some embodiments, a bias can be introduced to the similarity measure between possible trees and the minimal spanning tree to increase or decrease the semantic distances represented in the updated word set relative to the initial word set, and with that, increase or decrease the level of difficulty of the AVLT.

In alternative embodiments, the step of finding the minimal spanning tree can be avoided and the graph representing the words in the database can be searched for subgraphs representing a set of weighted edges that is similar to the graph formed by the words in the initial set. However this approach is more computationally expensive.

More generally, as a summary of the above techniques, step 105 can comprise forming an initial weighted graph from the words in the initial set and the semantic distances. The semantic distances form weights along edges of the graph and the words form vertices of the graph. The minimal spanning tree of the initial weighted graph is found (those skilled in the art will be aware of suitable techniques for finding a minimal spanning tree). A database weighted graph is formed from the words in the database and semantic distances between each pair of words in the database. A subtree is identified in the database weighted graph that has the desired level of similarity to the minimal spanning tree of the initial weighted graph, and one or more words can be extracted from the database according to the identified subtree in the database weighted graph.

In these embodiments, the desired level of similarity can be a required difference in a number of vertices between a subtree in the database weighted graph and the minimal spanning tree of the initial weighted graph, and a required difference in a distribution of weighted edges between a subtree in the database weighted graph and the minimal spanning tree of the initial weighted graph.

In a third set of embodiments (which can be used separately or in combination with the first set of embodiments or the second set of embodiments), the set of words is optimized (i.e. made harder, easier or maintained at a similar difficulty level) using personal information about the first subject. This information can be cultural information, sociodemographic information and/or information about comorbidities. It is known that personally relevant information is easier to memorize than information that is not personally relevant. For example, personally relevant information for someone growing up in the Beatles era would be words related to lyrics of Beatles songs. By changing the personal relevance of the words in the set, the difficulty level of the AVLT can be adapted (e.g. the difficulty level can be increase if the words are less personally relevant, and vice versa).

These embodiments can use as an additional input to the method various characteristics of the first subject, which can originate from various sources, for example a hospital's electronic medical record (EMR), personal health records (PHR), and potentially social media and other sources of personal preferences or personal information, such as buying patterns from online stores.

The characteristics can be used to form (or can be part of) a user profile for the first subject, for example by creating a vector representation where each entry corresponds to a characteristic such as age, gender, ethnicity, income level, education level, music preference, hobbies, etc. Previously-performed tests by a population of subjects can be used to derive a difficulty level per unique word, for example by counting the number of times a word has not been recollected or recalled during an AVLT. By aggregating the information over multiple subjects, it is possible to derive, for example through regression or machine learning techniques, a relationship between AVLT word difficulty and user profiles. It is possible to group together, for example using clustering techniques, similar user profiles and pairs of words and difficulty. This information can be used for the generation of an updated word set in several different ways. The first way is to randomly, given similarity of difficulty level, select from the words represented in the cluster closest to the first subject's user profile using a technique similar to the first set of embodiments above. In a second way, ontologies can be used that link user profile characteristics interpreted as word concepts to new words in the database. For example, if a user has a preference for classical music, an ontology will indicate that words representing symphonic musical instruments (such as a violin, cello, piano) and words representing classical music formats (such as piano concerto, symphony, sonata) are close to the concept of classical music, while more popular music terms (such as groove, hip-hop, heavy metal, rap) are more distant. These ontology-based distances can be used in a similar way as described in the second set of embodiments to generate updated word sets.

Thus, in general embodiments (i.e. not restricted to the first set of embodiments or the second set of embodiments), the method can further comprise the step of storing a results database that comprises results for AVLTs previously-performed by a plurality of subjects. The stored results can indicate whether the words in the AVLTs were successfully recalled by the relevant subject. The results may also indicate which words were in the set of words used for the AVLT, and which were recalled correctly or missed. Respective user profiles can also be stored for the plurality of subjects, with each user profile indicating one or more user characteristics, such as age, gender, ethnicity, income level, education level (e.g. high school, university degree, etc.), music preference, hobbies, etc. The results and user profiles can be stored in the memory unit 10, or a separate memory unit.

In some embodiments, the desired level of similarity to use in step 105 can be determined based on the one or more results for the first subject.

The method can include the additional step of analyzing the stored results and respective user profiles to determine a relationship between successful recall of a word and user profiles. Then, in step 105, one or more words can be extracted from the database based on the desired level of similarity, a user profile of the first subject and the determined relationship.

In alternative embodiments, step 105 comprises using an ontology to identify one or more words in the database for the first subject based on the first subject's user profile; and extracting one or more words from the database based on the desired level of similarity and the ontology-identified one or more words.

Therefore, there is provided techniques for determining an updated set of words for an to help mitigate practice effects associated with an individual repeating an with the same set of words.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

**Claims**

1. A computer-implemented method of determining an updated set of words for use in an auditory verbal learning test, AVLT, on a first subject, the method comprising:

   receiving (101), by a processing unit, an initial set of words for use in an AVLT, wherein the initial set comprises a predetermined number of a plurality of words stored in a database;
   processing (103), by the processing unit, the initial set to determine feature values for the initial set;
   extracting (105), by the processing unit, one or more words from the database based on a desired level of similarity between feature values associated with the one or more extracted words and the feature values of the initial set; and
   selecting (107), by the processing unit, one or more of the extracted words to include in an updated set of words for use in the AVLT for the first subject.

2. A method as defined in claim 1, wherein the feature values for the initial set comprise a respective concreteness score for each word that represents a level of abstractness of a concept represented by the word, a number of characters in the word, a number of vowels and/or a number of consonants in the word, a number of syllables in the word, an originating language of the word or a frequency of use of the word in text.

EP 3 682 796 A1

3.  A method as defined in claim 1, wherein the feature values for the initial set comprise distances between pairs of words in the initial set.

4.  A method as defined in claim 3, wherein the step of processing (103) the initial set to determine the feature values comprises:
    determining the distance between each pair of words using an ontology.

5.  A method as defined in any of claims 1-4, wherein the method further comprises:
    storing, in a memory unit, a results database comprising results for AVLTs previously-performed by a plurality of subjects, and respective user profiles for the plurality of subjects, wherein the results indicate whether the words in the AVLTs were successfully recalled by the subject.

6.  A method as defined in claim 5, wherein the results database comprises one or more results for the first subject, and wherein the method further comprises:
    determining the desired level of similarity based on the one or more results for the first subject.

7.  A method as defined in claim 5 or 6, wherein the method further comprises:
    analyzing the stored results and respective user profiles to determine a relationship between successful recall of a word and user profiles.

8.  A method as defined in claim 7, wherein the step of extracting (105) comprises:
    extracting one or more words from the database based on the desired level of similarity, a first user profile of the first subject and the determined relationship.

9.  A method as defined in claim 5 or 6, wherein the step of extracting (105) comprises:

    using an ontology to identify one or more words in the database for the first subject based on a first user profile for the first subject; and
    extracting one or more words from the database based on the desired level of similarity and the ontology-identified one or more words.

10. A computer-implemented method of administering an auditory verbal learning test, AVLT, to a first subject, the method comprising:

    determining an updated set of words for use in an AVLT according to any of claims 1 to 9; and
    outputting, via a user interface, the updated set of words to the first subject.

11. A computer program product comprising a computer readable medium having computer readable code embodied therein, the computer readable code being configured such that, on execution by a suitable computer or processor, the computer or processor is caused to perform the method of any of claims 1-10.

12. An apparatus (2) for determining an updated set of words for use in an auditory verbal learning test, AVLT, on a first subject, the apparatus (2) comprising:
    a processing unit (8), wherein the processing unit (8) is configured to:

    receive an initial set of words for use in an AVLT, wherein the initial set comprises a predetermined number of a plurality of words stored in a database; and
    process the initial set to determine feature values for the initial set;
    extract one or more words from the database based on a desired level of similarity between feature values associated with the one or more extracted words and the feature values of the initial set; and
    select one or more of the extracted words to include in an updated set of words for use in the for the first subject.

13. An apparatus (2) as defined in claim 12, wherein the feature values for the initial set comprise a respective concreteness score for each word that represents a level of abstractness of a concept represented by the word, a number of characters in the word, a number of vowels and/or a number of consonants in the word, a number of syllables in the word, an originating language of the word or a frequency of use of the word in text.

14. An apparatus (2) as defined in claim 12, wherein the feature values for the initial set comprise distances between pairs of words in the initial set.

15. An apparatus (2) as defined in any of claims 12-14, wherein the apparatus (2) is further for administering the AVLT to the first subject, and the apparatus (2) further comprises a user interface (14) that is for outputting the updated set of words to the first subject.

Fig. 1

EP 3 682 796 A1

Fig. 2

Europäisches
Patentamt
European
Patent Office
Office européen
des brevets

# EUROPEAN SEARCH REPORT

Application Number

EP 19 15 2473

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2017/177826 A1 (ALEXANDER GREGORY E [US] ET AL) 22 June 2017 (2017-06-22) * paragraph [0033] - paragraph [0035] * ----- | 1-15 | INV. A61B5/00 G16H50/20 G06F16/33 |
| | | | TECHNICAL FIELDS SEARCHED (IPC) |
| | | | A61B G16H G06F |

The present search report has been drawn up for all claims

| Place of search | Date of completion of the search | Examiner |
|---|---|---|
| Munich | 15 May 2019 | Herri, Edmond |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 19 15 2473

This annex lists the patent family members relating to the patent documents cited in the above-mentioned European search report.
The members are as contained in the European Patent Office EDP file on
The European Patent Office is in no way liable for these particulars which are merely given for the purpose of information.

15-05-2019

| Patent document cited in search report | Publication date | Patent family member(s) | Publication date |
|---|---|---|---|
| US 2017177826 A1 | 22-06-2017 | US 2017177826 A1<br>WO 2015120481 A1 | 22-06-2017<br>13-08-2015 |

EPO FORM P0459

For more details about this annex : see Official Journal of the European Patent Office, No. 12/82